# EUROPEAN PATENT APPLICATION

(11) **EP 4 362 033 A1**
(43) Date of publication of application: **01.05.2024**
(21) Application number: 22203204.7
(22) Date of filing: 24.10.2022
(51) Int. Cl.: G16H 10/60, G06N 3/08, G06N 20/00, G10L 15/00, G16H 40/20

(54) **PATIENT CONSENT**

(71) Applicant: Koninklijke Philips N.V., 5656 AG Eindhoven (NL)
(72) Inventor: HENDRIKS, Monique, Eindhoven (NL); TALGORN, Elise Claude Valentine, Eindhoven (NL); BUIL, Vincentius Paulus, 5656AG Eindhoven (NL)
(74) Representative: Philips Intellectual Property & Standards

(57) **Abstract**

A means for assessing consent of a patient via automated analysis of the audio data of a conversation between a patient and clinician in which various items for which patient consent is needed are discussed. Natural language processing can be used to detect when particular consent items are discussed, and to determine a confidence score in respect of one or more of: sufficiency of explanation, sufficiency of understanding and provision of consent by the patient.

## Description

### FIELD OF THE INVENTION

This invention relates to determining patient consent via natural language processing.

### BACKGROUND OF THE INVENTION

In medical context, consent is required from patients for many things. These include, by way of non-limiting example: agreeing to data sharing; agreeing to receive a diagnostic test; agreeing to receive a treatment or (tele)therapy or procedure; acknowledging an understanding of possible risks and complications; acknowledging an understanding of a nature of the treatment or procedure; acknowledging an understanding that certain actions should be avoided e.g. drink or eat a certain number of hours before the procedure; confirming that provided information is accurate; confidentiality clauses; cancelation/rescheduling clauses; and insurance clauses.

The process of obtaining consent from a patient can be cognitively demanding for a patient. This is even more so if the patient is very unwell and therefore not in an ideal state to, for example, read and understand long consent forms and deliberate on their response. Also, from the perspective of the practitioner, it is time-consuming and repetitive.

### SUMMARY OF THE INVENTION

The invention is defined by the claims.

According to examples in accordance with an aspect of the invention, there is provided a computer-implemented method. The method comprises communicating with a datastore to access a data list reciting a plurality of consent items, each consent item corresponding to an element of patient care which requires patient consent.

The method further comprises receiving audio data of the whole or part of a patient-clinician conversation.

The method further comprises processing the audio data with a speech analysis module. The speech analysis module is configured to: apply automated speech recognition to convert the audio data to a textual representation of the conversation; and apply natural language processing to the textual representation of the conversation to detect at least a portion of the conversation in which at least one of the consent items is discussed.

For each said at least portion of the conversation, the method further comprises applying a consent assessment comprising applying natural language processing to determine one or more of: a confidence score relating to a sufficiency of explanation by the clinician of the consent item discussed; a confidence score relating to a sufficiency of understanding by the patient of the consent item discussed; and a confidence score relating to a confidence that consent has been given by the patient in relation to the consent item.

The method preferably further comprises generating a data output for communication to a user interface device based on the consent assessment.

Thus, embodiments of the present invention propose to automatically process an audio data feed of a conversation between a patient and a clinician, to automatically detect if and when each of a pre-determined list of consent items (being items for which patient consent is needed) are discussed, and then automatically processing a text-transcribed version of each portion of the conversation related to each consent item to make one or more assessments of the degree and quality of consent given by the patient. The assessment comprises use of natural language processing, and seeks to derive one or more confidence scores relates to the quality of explanation by the clinician, the degree of comprehension by the patient, and the confidence that a patient gave consent as part of the conversation. This method enables consent acquisition to be performed in a much more natural way, without paper forms. Furthermore, the one or more confidence scores allow for quality of consent to be assessed (which is simply not possible with a standard paper form), and, if the scores are too low, in some embodiments, feedback can be provided to the clinician and the clinician can supplement the conversation with additional explanation, additional checks of patient understanding, and questions seeking further positive confirmation by the patient of their consent.

In some embodiments, the analysis module is adapted to apply natural language processing to the textual representation of the conversation to detect discussion or presence in the conversation of one or more of a pre-defined list of topics and/or statements recorded in a topic database.

In some embodiments, the topic database further records for each of the pre-defined list of topics and/or statements one or more of the consent items to which the topic and/or statement is relevant.

In some embodiments, the method comprises relating each of the topics and/or statements detected in the conversation to one or more of the consent items using the topic database. In this way, the conversation can be analyzed to detect when particular consent items in the list are discussed, by virtue of detecting and analyzing the topics that are discussed, and using the relevance links stored in the topic database between different topics and different consent items.

In other words, in some embodiments, the process of determining the at least portion of the conversation in which at least one of the consent items is discussed is performed based on analysis of the detected topics and/or statements which have been detected as related to the given consent item.

In some embodiments, the process of determining the one or more confidence scores for a given consent item is additionally or alternatively performed based on analysis of the detected topics and/or statements which have been detected as related to the given consent item.

In some embodiments, the one or more confidence scores are determined based on evaluation of one or more pre-defined criteria relating to the topics and/or statements detected as relevant to the given consent item. Options relating to such criteria will be discussed later in more detail.

In some embodiments, the determining the one or more confidence scores is performed based on counting the number of topics and/or statements which have been detected as related to the given consent item for which the score is being determined. For example the confidence score may be computed in correlation with this count.

In some embodiments, the determining the confidence score relating to a sufficiency of explanation includes counting how many of a pre-defined list of topics and/or statements relating to the given consent item are present in the at least portion of the conversation. For example the confidence score may be computed in correlation with this count.

In some embodiments, the determining the confidence score relating to a sufficiency of explanation or sufficiency of understanding includes mapping of detected topics and/or statements present in the at least portion of the conversation to one or more logic rules to assess whether the one or more logic rules are met. This will be explained with greater detail to follow. In summary, the patient's understanding can be assessed by determining how well the patient's restatement of certain consent items matches certain formal or semantic logic rules. This can assess how well they have understood. The same principle can be applied to assessing the explanation of the clinician. If the clinician has explained the consent item well, their explanation should meet certain logic rules or patterns.

In some embodiments, the consent assessment can be performed in real time while the conversation is ongoing so that feedback can be given during or immediately after the conversation. This allows for any insufficiency in explanation, understanding, consent, or any other indicators to be remedied by augmenting the conversation.

Thus, in some embodiments, the method comprises processing the audio data in real time, and providing feedback indicative of results of the consent confidence assessments in real time to the clinician.

In some embodiments, the speech analysis module is configured to process the audio data in real time and to detect, based on the processing, when discussion of any of the list of consent items begins, and responsive thereto trigger start of a recording of the discussion. For example, this can be stored (temporarily) in a database in case the doctor needs to or wants to refer back to it later in the conversation.

In some embodiments, following the start of said recording, the speech analysis module may be configured to detect, based on the processing, when the discussion of the given consent item ceases. Responsive to detecting this, the speech analysis module may be configured to stop the recording of the discussion, to provide a recording of the at least portion of the conversation relating to the given consent item. The speech analysis module may further be configured to apply the consent assessment to said at least portion of the conversation covered by the recording.

For example, it may be detected that the discussion of the consent item is stopped based on detecting that the patient has consented or rejected or by detecting that the discussion has moved on to another topic.

In some embodiments, the method may comprise comparing each of the one or more confidence scores of the consent assessment against a respective threshold. In some embodiments, the method may comprise generating an output for communication to a user interface, the output being based on a result of the comparison. This might be provided in real time, during or immediately after the conversation for example. This way, if the confidence scores fail to meet the threshold, the clinician can supplement the conversation with further explanation.

In some embodiments, the consent assessment, the score comparison and the output generation are triggered immediately responsive to said detection of the cessation of the discussion of the given consent item of the at least portion of the conversation relating to the given consent item. This therefore provides an immediate feedback loop to the clinician, allowing them to add to the conversation to improve any of the confidence scores which fail to meet the given threshold. Thus, in operation, the experience of the clinician and patient would be one of a natural conversation which flows from discussion of one consent item to the next, but wherein, while the conversation is ongoing, a user interface (probably visible to the clinician for example) displays live results of the consent assessment as the discussion moves on from one item to the next.

In some embodiments, the speech analysis module may be further configured to detect an end of the conversation, and wherein the consent assessment further comprises detecting whether consent has not been given for any consent items in said list of consent items, and providing feedback to the clinician via a user interface indicative of this detection. Thus, some or all of the feedback might be provided at an end of the conversation, in addition to, or instead of, providing the feedback in real time as each consent item is discussed. If this is done instead of the real-time feedback, this may avoid for example interrupting the flow of the conversation and disturbing the thought process of the clinician.

In some embodiments, the method may further comprise, for each consent item for which it has been detected that consent has not been given, retrieving the recording of the at least portion of the conversation relating to said consent item or the textual representation of the at least portion of the conversation relating to said consent item; and providing via a user interface a control option permitting replaying of the recording, or permitting display of the textual representation.

Thus, feedback at the end of conversation may comprise parts of the conversation associated with the consent item being recalled by the system and presented back to the doctor and patient for review. Optionally, in some embodiments, more information on the consent item may be presented by the system to supplement the doctor's explanation of the item.

In some embodiments, the method further comprises communicating with a patient database, and accessing a patient record for the patient. The patient record may include an indication of one or more planned elements of patient care for the patient. The method may comprise generating the original list of consent items based on application of a lookup table which maps elements of patient care to consent items.

In some embodiments, the determining the consent items could be performed partially predictively. For example, it could be partly done through analyzing future appointments or elements of the future care path that can be obtained directly from the patient's medical file. Partly this could be done through predicting the future care path by comparing the patient to similar patients who have already been treated in the past, or by mapping the patient's situation onto an existing protocol or guideline for treatment.

In some embodiments, the method further comprises receiving input camera image data from a camera arranged to record the patient's face and/or body during the conversation.

In some embodiments, the method further comprises processing the camera image data with a facial expression and/or body language processing module;

In some embodiments, the determining of the confidence score relating to a sufficiency of understanding by the patient of the consent item discussed further comprises use of an output from the facial expression and/or body language processing module.

In other words body language and/or facial expressions may be evaluated and linked to parts of the conversation to assess confidence in the patient's comprehension.

In some embodiments, brain activity patterns could also be measured and used to estimate the level of concentration that the patient has on the conversation, and wherein the detected level of conversation is used in determining the confidence score relating to a sufficiency of understanding by the patient of the consent item.

In some embodiments, the method further comprises communicating with a patient consent database storing a record of consent items for which consent has been given by the patient, and updating the database to reflect consent items for which the consent assessment is positive.

In some embodiments, the method further comprises communicating with a patient consent database storing a record of consent items for which consent has been given by the patient, and wherein the list of consent items is modified to remove any consent items for which said patient consent database indicated consent has already been given. In other words, the system in some embodiments can keep track of what consent was already given in conversations with other health care professionals and informs the doctor in the current conversation which items do not require consent anymore and which do.

In some embodiments, said patient consent database may be a centrally accessible database, accessible by multiple care institutions. For example, consent registration is stored in a personal health record, or some other centrally stored record, such that it can be shared between care institutes. This would improve efficiency.

The invention can also be embodied in software form. One aspect of the invention provides a computer program product comprising code means configured, when run on a processor, to cause the processor to perform a method in accordance with any embodiment or example described in this disclosure or in accordance with any claim of this application.

The invention can also be embodied in hardware form. Thus, another aspect of the invention is a processing unit. The processing unit comprises an input/output and/or communication interface, and one or more processors, The one or more processors may be configured to perform a method in accordance with any of the embodiments described in this disclosure or in accordance with any claim of this application. For example, the one or more processors may be configured to communicate, via the input/output, with a datastore to access a data list reciting a plurality of consent items, each consent item corresponding to an element of patient care which requires patient consent. The one or more processors may be further adapted to receive audio data of the whole or part of a patient-clinician conversation. The one or more processors may be further adapted to process the audio data with a speech analysis module, wherein the speech analysis module is configured to: apply automated speech recognition to convert the audio data to a textual representation of the conversation; and apply natural language processing to the textual representation of the conversation to detect at least a portion of the conversation in which at least one of the consent items is discussed. The one or more processors may be further configured to, for each said at least portion of the conversation, apply a consent assessment comprising applying natural language processing to determine one or more of: a confidence score relating to a sufficiency of explanation by the clinician of the consent item discussed; a confidence score relating to a sufficiency of understanding by the patient of the consent item discussed; and a confidence score relating to a confidence that consent has been given by the patient in relation to the consent item. The one or more processors may be further configured to generate a data output for communication to a user interface device based on the consent assessment, and route the data output to the input/output.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 outlines steps of an example method in accordance with one or more embodiments of the invention;
Fig. 2 schematically outlines components of an example processing arrangement in accordance with one or more embodiments of the invention;
Fig. 3 provides a visual representation of content of an example topic database for use by a speech analysis module according to one or more embodiments of the invention;
Fig. 4 schematically outlines in more detail an example processing flow according to one or more embodiments of the invention; and
Fig. 5 schematically outlines a system architecture and processing flow according to a set of one or more embodiments of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a means for assessing consent of a patient via automated analysis of the audio data of a conversation between a patient and clinician in which various items for which patient consent is needed are discussed. Natural language processing can be used to detect when particular consent items are discussed, and to determine a confidence score in respect of one or more of: sufficiency of explanation, sufficiency of understanding and provision of consent of the patient.

The proposed system and method thus allow for keeping track of and guiding verbal consent for medical interventions in a conversation with a doctor.

In some proposed embodiments, the method comprises analyzing the conversation between doctor and patient and mapping their speech onto consent items from the patient's care path, or stored in another data source. Through analyzing the response of the patient, a confidence level can be attached to whether the patient has understood the consent item and does truly agree to it.

In some advantageous embodiments, by also providing feedback in real time regarding the patient understanding, this allows the doctor to explain consent items in a way that addresses the educational level and communication preferences of the individual patient.

In some advantageous embodiments, by also mapping the consent items onto the patient's care path, superfluous consent items may be omitted from the process.

A general advantage is that the standard consent process comprising a patient reading and completing a long consent form can be replaced by a verbal stepwise consent process, in which items are discussed and the patient's giving of consent or not is automatically detected and recorded.

However, it has been realized by the inventors that a potential new problem introduced by this solution is that such a way of obtaining consent might be more error prone. In a conversation, the doctor may easily forget to mention certain items, or phrase them in a way that leads the patient to misinterpret them. In writing, the patient can read the item several times, while when spoken, the patient must understand it the first time or ask for the clinician to repeat. This can create a barrier to good understanding. Furthermore, the answer of the patient might be unclear, i.e. not a simple yes/no answer. Finally, the speech recognition system may be susceptible to errors, especially in the case of complex answers or voices that are difficult to recognize such as elderly, some sick patients, or patients speaking with a strong accent.

These potential new problems are addressed by embodiments of the present invention through the generation of one or more confidence scores relating to one or more aspects of the consent discussion.

In particular, items that have been discussed are assigned a level of confidence, indicating one or more of:
to what extent the system is confident that the relevant item was explained well;
to what extent the system is confident that the item was understood by the patient;
to what extent the system is confident that consent was given by the patient.
In preferred embodiments, all three of these confidence scores are computed. However, a beneficial technical effect is achieved even if only a subset is computed.

In a preferred set of embodiments, at the end of the conversation, items that have not been discussed or for which consent was not obtained from the patient are presented to the clinician and/or patient to make sure that consent is obtained for all items. In some embodiments, parts of the conversation associated with the consent item may be recalled by the system and presented back to the doctor and patient for review. In some embodiments, more information on the consent item may be presented by the system to supplement the doctor's explanation of the item.

In some embodiments, functionality is included to enable tailoring of consent to the individual patient. For example, only consent items which are potentially relevant for the future treatment of the patient are assessed as part of the consent assessment, while other consent items can be left out. In case the foreseen care path of the patient changes, and e.g. additional diagnoses or treatments are planned, any consent items previously omitted might be revisited and included at a later stage.

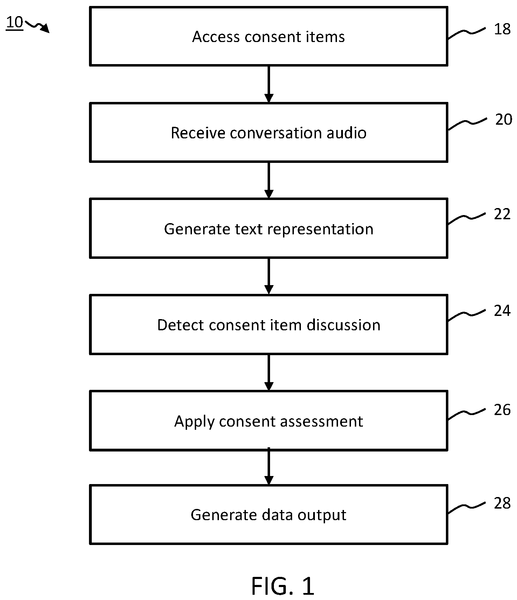

Fig. 1 outlines in block diagram form steps of an example method according to one or more embodiments. The steps will be recited in summary, before being explained further in the form of example embodiments. The method may be implemented by a computer or processor in some embodiments.

The method comprises communicating with a datastore to access 18 a data list reciting a plurality of consent items, each consent item corresponding to an element of patient care which requires patient consent. For example, each consent item may correspond to a proposed treatment or investigation or other intervention, surgical, pharmaceutical, or otherwise. The datastore may be a local or remote datastore. It may contain a patient database. The list of consent items may be patient specific. For example, there may be stored in the datastore a respective list of consent items for each of a plurality of patients and wherein the datastore is queried or interrogated based on patient identification information to obtain a consent item list for the relevant patient.

The method further comprises receiving 20 audio data of the whole or part of a patient-clinician conversation. This may be received for example from an audio recording apparatus, for example comprising one or more microphones. This may be a dedicated audio recording apparatus or may be implemented using for example recording apparatus integrated in a mobile computing device such as a smartphone. In the conversation, it is assumed that the clinician and patient are discussing the different consent items. Alternatively, the audio data might be received from a datastore or remote computer which stores audio data of a previous conversation.

The method further comprises processing the audio data with a speech analysis module. The analysis module is configured to apply automated speech recognition to generate 22 a text representation of the conversation. In other words, the automated speech recognition converts the audio data to a textual representation of the conversation. The speech analysis module is further configured to apply natural language processing to the textual representation of the conversation to detect 24 at least a portion of the conversation in which at least one of the consent items is discussed.

For each at least portion of the conversation which is so detected, the method further comprises applying 26 a consent assessment comprising applying natural language processing to determine one or more consent-related confidence scores. In particular, these may include one or more of: a confidence score relating to a sufficiency of explanation by the clinician of the consent item discussed; a confidence score relating to a sufficiency of understanding by the patient of the consent item discussed; and a confidence score relating to a confidence that consent has been given by the patient in relation to the consent item.

The method may further comprise generating a data output, for instance for communication to a user interface device based on the consent assessment and/or for communication to a datastore for recording the consent assessment results.

As noted above, the method can also be embodied in hardware form.

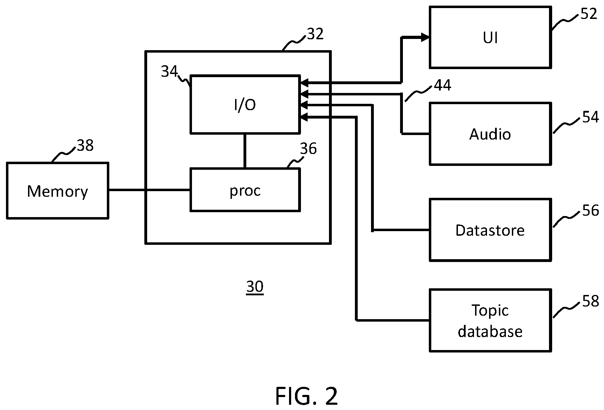

To illustrate, Fig. 2 presents a schematic representation of an example system 30 in accordance with one or more embodiments of the invention.

The illustrated system comprises a processing unit 32 comprising an input/output 34 or communication interface and one or more processors 36.

The one or more processors 36 are configured to perform a method in accordance with any example or embodiment described in this document, or in accordance with any claim of this application. For example, the one or more processors may perform the method as outlined above.

The processing unit 32 may be provided alone as an aspect of the invention, since this alone embodies the inventive concept by virtue of being programmed to perform the method.

Another aspect is a system 30 which comprises the processing unit 32 in addition to one or more further elements. For example, the system may comprise a user interface (UI) device 52. The system 30 may comprise an audio recording apparatus 54 for recording the audio data 44 of the conversation. The system may comprise the datastore 56 which stores the at least one list of consent items. The system may comprise a topic database which stores associations between different conversational topics detectable by the natural language processing and a variety of different possible consent items that may appear in the list. More details relating to this feature will be described later. The system may comprise all of the aforementioned elements, or a subset, or none. In some embodiments, the system may further comprise a memory module 38 which stores for example computer program code which is executable by the one or more processors 36 to cause the execution of the relevant method.

Indeed, the invention can also be embodied in software form. Thus another aspect of the invention is a computer program product comprising code means configured, when run on a processor, to cause the processor to perform a method in accordance with any example or embodiment outlined in this document, or in accordance with any claim of this patent application.

As has been briefly mentioned above, in order to perform the detection of the discussion of the different consent items, use may be made of a topic database which stores a set of associations between particular possible consent items and different topics/statements that might be discussed. These different topics/statements might be further broken down into sub-topics and sub-sub-topics and so on in a branching structure. In operation, the natural language processing may be configured to detect topics and/or statements and then use the topic database to link these to particular consent items to which those topics/statements are relevant.

In other words, the speech analysis module may adapted to apply natural language processing to the textual representation of the conversation to detect discussion or presence in the conversation of one or more of a pre-defined list of topics and/or statements recorded in a topic database 58, and wherein the topic database further records for each of the pre-defined list of topics and/or statements one or more of the consent items to which the topic and/or statement is relevant. Indeed, the structure may be the reverse in some cases, wherein the topic database records a set of consent items, and further records for each consent item a set of different topics/statements linked or associated with that consent item. This can be understood conceptually as the topic database containing one or a plurality of topic maps relating consent items to certain concepts or topics or statements that may arise during the conversation between doctor and patient.

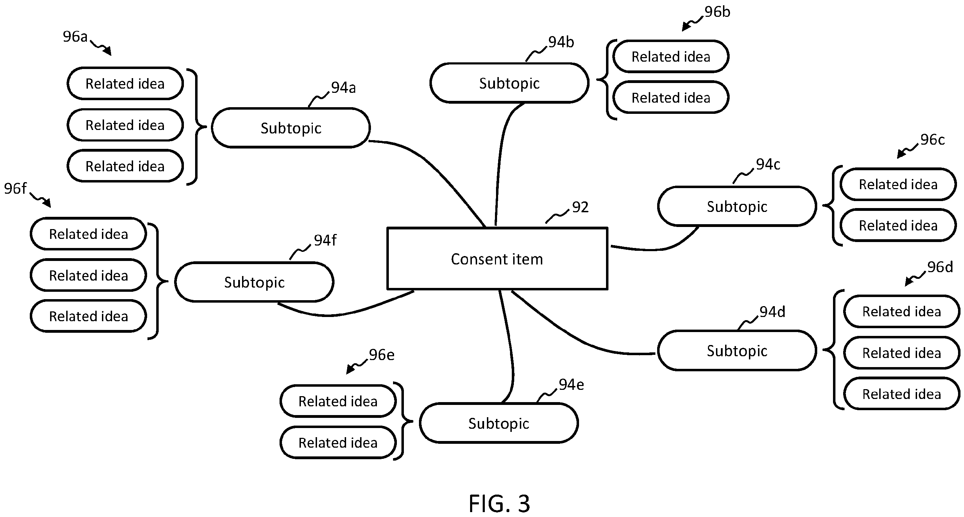

By way of schematic illustration, Fig. 3 presents a visual representation of the type of content which the topic database might store. The central node 92 in this case may represent a single one of the possible consent items, for example: sharing data regarding medication use. Linked to it are a plurality of topics or subtopics 94 which relate to this consent item. For example, each could relate to specific medications or brands of medication that the patient uses or has used in the past, pharmacies where the patient has picked up prescriptions, etc. Each of these sub-topics 94 could be even further broken down, wherein each is linked with a respective set of sub-subtopics or related ideas 96.

Thus, it can be understood that the topic database could be initially built up through reference to data stored in a patient record in a patient database, e.g. relating to medications, medical history, clinical condition, planned interventions, clinicians involved in patient's care and so on. The topic database may thus be patient-specific. There may be an initialization process in which the topic database is constructed for the patient in advance of the method of recording and processing the conversation. This process might be based on an initial template or shell topic database, in which template items are then populated based on data in the patient database. For example, the template or shell topic database might include fields relating to patient medication, medical history, clinical condition and any other relevant topics and/or concepts, and these might be filled in or populated based on querying similar fields in the patient's record in the patient database.

If these topics 94 arise in the conversation, then it is possible that the patient and clinician are discussing the consent item of sharing data regarding medication use. Thresholding may be applied such that once a certain number of the topics or subtopics associated with a particular consent item have been detected by the speech analysis module, then it is determined that the particular consent item is being discussed. Another possible criterion could be that if a threshold proportion of all of the topics/statements detected during a particular period are linked to a given consent item, then it is determined that the said consent item is being discussed during that period.

In other words, and to state the concept in more general terms, the method in some embodiments comprises relating each of the topics and/or statements detected in the conversation by the speech analysis module to one or more of the consent items using the topic database 58.

Then, the determining the at least portion of the conversation in which at least one of the consent items is discussed may be done based on analysis of the detected topics and/or statements which have been detected as related to the given consent item. As mentioned above, this could be done using thresholding criteria of different kinds.

Also, the determining the one or more confidence scores for a given consent item can also be performed using the topic database. For example, the one or more confidence scores might be determined for a given consent item based on evaluation of one or more pre-defined criteria relating to the topics and/or statements detected as relevant to the given consent item during the portion of the conversation when the consent item is detected as having been discussed. Examples of criteria which may be applied will be explained in further detail to follow.

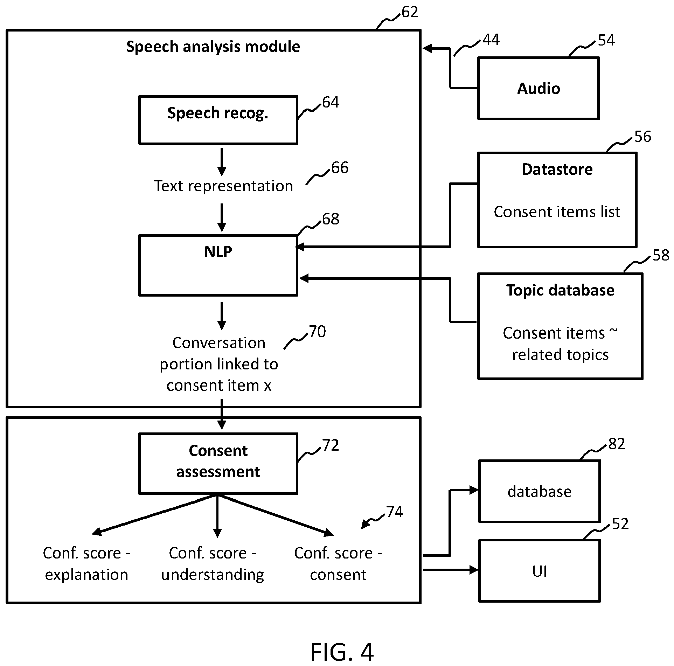

Before discussing in more detail how the different confidence scores of the consent assessment may be determined, the processing flow discussed above will be further illustrated with reference to Fig. 4. Fig. 4 schematically outlines the processing flow according to one or more embodiments.

As illustrated a speech analysis module 62 receives audio data 44 of a patient-clinician conversation, for example from an audio recording apparatus 54. Automated speech recognition 64 is applied to convert the audio data to a textual representation 66 of the conversation. As previously discussed, connection is made with a datastore 56 to access a data list reciting a plurality of consent items, each consent item corresponding to an element of patient care which requires patient consent. Natural language processing 68 is applied to the textual representation of the conversation to detect at least a portion 70 of the conversation in which at least one of the consent items is discussed. As previously discussed, this may make use of a topic database 58 which links different possible consent items with a set of related topics and/or statements which might arise in the course of the conversation.

For each said at least portion of the conversation which is detected, a consent assessment 72 procedure is applied which comprises applying natural language processing to determine one or more confidence scores 74 related to consent. The one or more confidence scores may include: a confidence score relating to a sufficiency of explanation by the clinician of the consent item discussed; a confidence score relating to a sufficiency of understanding by the patient of the consent item discussed; and/or a confidence score relating to a confidence that consent has been given by the patient in relation to the consent item. Feedback related to the result of the consent assessment may then be communicated to a user interface device 52 for display on the user interface. Additionally or alternatively, the results of the consent assessment or information related thereto may be output to a database 82. In Fig. 4, this is shown as a separate database, but it could in other cases be the datastore 56 storing the consent items or a patient database storing patient records.

One part of the method includes the use of natural language processing to detect discussion of the different consent items, and wherein this may include application of natural language processing to detect discussion or present in the conversation of one of a pre-defined list of topics and/or statements in a topic database.

By way of further explanation, application of natural language processing (NLP) to detect topics or concepts in a text representation of a discussion is a task which can be performed using known NLP analysis techniques. Topic analysis is a known NLP technique to automatically detect topics in text. In particular, a machine learning or artificial intelligence algorithm could be used to perform topic analysis. Two common approaches for topic analysis are NLP topic modelling and NLP topic classification. For embodiments of the present invention, NLP text classification may provide a more effective approach. This allows for extraction of topics from text, where the particular topics to be detected are defined in advance. For embodiments of the present invention, the relevant topics and/or statements to be detected may be defined in a topic database 58, as has already been discussed. Topic detection can be based on detection of particular patterns of keywords located with a certain proximity to one another for example.

In some embodiments, a machine learning algorithm might be employed which is trained to detect one or more of the topics. Suitable machine learning algorithms include for example a convolutional neural network, a naive Bayes algorithm, a support vector machine, or a recurrent neural network. The machine learning algorithm may be trained in a supervised training procedure. A training dataset used for the training might comprise a plurality of sample text conversations which are annotated/labelled according to particular topics/statements contained in the text. The machine learning algorithm can then be trained through applying the algorithm to each sample text conversation, and comparing the output against the annotated ground truth, and with the weightings of the neural network being adjusted each time to minimize a loss function for example. By way of example, the machine learning algorithm may comprise an encoder layer for converting input text into corresponding feature vectors using standard text vectorization techniques, and include one or more layers for generating topic predictions based on processing of the feature vectors.

Use of a machine learning algorithm however is not essential. It is also possible to use a rules-based algorithm to perform the NLP topic detection. A rules-based algorithm works by directly programming particular rules by which different topics or statements of interest can be detected. The rules may each consist of a certain pattern to be detected in the text (e.g. a combination of keywords, within a certain proximity or relationship to one another), where this pattern is associated with a particular topic.

As previously discussed, one part of the method is a consent assessment 72. Further implementation details in accordance with one or more possible embodiments related to the consent assessment will now be discussed.

The consent assessment includes generating at least one of three confidence scores, which respectively estimate how likely it is that:
The doctor has explained the consent item correctly and comprehensively;
The patient has interpreted the consent item correctly;
The patient has given consent.

In some embodiments, computing each of the three confidence assessment components 74 may employ use of use of machine learning to generate the respective confidence scores.

Different embodiments are possible in respect of the computation of each score.

As a general principle, the determining the one or more confidence scores may be performed based on assessment of the topics and/or statements (see discussion above relating to the topic database) which have been detected as related to the given consent item for which the score is being determined.

In some embodiments, rules-based algorithms may be used to generate the confidence scores, based on analysis of the topics/statements detected in the conversation. Examples will now be discussed.

By way of example, some of the scores could be computed based on counting a number of topics and/or statements which have been detected as related to the given consent item for which the score is being determined. As has already been discussed, in identifying different portions of the conversation which relate to particular consent items, the speech analysis module 62 may use natural language processing to detect mentions of particular topics and/or statements listed in a topic database, and further use the topic database to relate these to particular consent items. In this way, it can be determined when a particular consent item is being discussed. The results of this processing operation already include an identified list of topics and/or statements which were mentioned during the relevant portion of the conversation which relate to the given consent item. Thus, the confidence scores can be further computed based on analysis of this list of identified topics and/or statements.

Starting with the confidence score relating to sufficiency of explanation, this is intended to provide an assessment of the degree to which the explanation provided by the doctor is correct and comprehensive. The speech analysis module 62 may process the identified list of topics and/or statements that have been mentioned by the clinician relating to the consent item to assess how accurately and comprehensively the consent item has been discussed. By way of one example, the consent item might be: asking consent to share data relating to past medication use. In this case, the topic database might list the following set of topics and/or concepts related to this consent item:
The concept of 'medication';
Different entities with whom data may be shared (e.g. initially populated based on reference to data stored in a patient database);
A description of what exactly will be shared;
A time period over which data may be shared (start date, end date).

The more of these concepts that are mentioned, the higher the confidence that the item has been discussed comprehensively.

In other words, and as a more general concept, the determining the confidence score relating to a sufficiency of explanation may include counting how many of a pre-defined list of topics and/or statements relating to the given consent item (as determined from the topic database) are present in the at least portion of the conversation.

With regards to the correctness of the explanation, this could be assessed through mapping the text analysis to a set of logic rules to which the explanation should comply.

In other words, and as a more general concept, it can be said that the determining the confidence score relating to a sufficiency of explanation may include mapping of detected topics and/or statements present in the at least portion of the conversation to one or more logic rules to assess whether the one or more logic rules are met.

By way of illustration, and continuing the above example consent item, an example of a logic rule that an explanation relating to sharing of medication data should comply with might be:
*If entity X is in the list of entities with whom medication data may be shared (as recorded in the topic database), and medication Y is in the list of medication that the patient uses (as recorded in the topic database), then any sentence containing X and Y should have a semantic breakdown into data sharing(X,Y)* = *YES.*

In other words, for this rule, any sentence mentioning X and Y and the relationship of data sharing should be a positive statement, reinforcing in the listener the belief that entity X can indeed get access to information Y.

By pre-storing a set of these rules, with one or more rules relating to each possible consent item, it would be possible to perform an at least partial assessment of the degree of accuracy of the clinician's explanation of the consent item.

The overall confidence score relating to sufficiency of explanation may be computed based on a combination of an assessment of both comprehensiveness and accuracy of explanation. Comprehensiveness could be assessed based on counting the number of relevant topics, statements and/or concepts which are mentioned (as discussed above) and accuracy might be assessed based on the assessment of compliance with the one or more logic rules (as also discussed above).

With regards to the confidence score relating to sufficiency of explanation, there are a number of possible approaches and options. As mentioned above, for the assessment, it is assumed that there have already been derived by the speech analysis module a list of the topics, concepts and/or statements related to the given consent item which have been mentioned in the particular potion of the conversation in which that consent item is discussed. This can then be processed and analyzed in different ways to assess confidence in the patient's understanding.

By way of one example, the more often a concept is mentioned by the doctor using the same or different particular wording, the higher the confidence that the patient correctly interprets it. For example, the word medication may be used, but also specific forms of medication such as pills, inhalers, sirops and even brands or types of medication such as beta-blockers or metoprolol (the most commonly prescribed beta-blocker).

The more times that these concepts are mentioned by the doctor, and with reference to the patient's specific circumstances, the higher the confidence that the patient correctly interprets them. For example the doctor may explicitly state that the patient's use of the beta-blocker metoprolol can be shared with the surgeon at hospital Z.

Additionally or alternatively, the method may involve detecting instances of the patient mentioning one or more of the topics and/or statements, and mapping the patient's mentions to one or more logic rules to assess a degree of accuracy of the patient's understanding. If the patient correctly repeats certain concepts, the confidence score may be increased.

Conversely, following a similar principle, if the patient incorrectly uses certain concepts, the confidence is decreased.

Furthermore, instances of the patient asking questions can be detected and assessment can be made of the questions posed, using NLP, to provide an indication of the degree of correct understanding or misunderstanding.

Examples have been discussed above in which one or more rules-based algorithms are applied to the already detected topics/statement present in the conversation to determine the one or more confidence scores. Additionally or alternatively, according to one or more embodiments, one or more machine learning algorithms may be employed for computing the confidence scores.

For example, the machine learning algorithm might be trained to receive as input the output of the speech analysis module 62, comprising data indicative of the detected topics/statements in the text version of the conversation. The machine learning algorithm may be trained to predict each of one or more of the confidence scores based on this input.

For example, for generating the confidence score relating to a sufficiency of explanation by the clinician of the consent item discussed, the machine learning algorithm might be trained as follows. A training dataset may include training data entries comprising sample outputs from the speech analysis module applied to sample conversations, comprising the detected set of topics/statements for each sample conversation. Each of these may be tagged or annotated in advance with a corresponding score indicating sufficiency of explanation. The annotations might be derived as follows. Each training data sample might be derived from conversations conducted as part of a study in which doctors provide different explanations to lay people and then the lay people receive a test to determine how well they understood the explanation.

In another embodiment, rather than having the lay people take a test, a third observer might rate the level of understanding of the patient. To improve robustness, a plurality of observers might rate the level of understanding of the patient and wherein an average is used as the confidence score annotation.

In a similar fashion, for the confidence score relating to a sufficiency of understanding by the patient of the consent item discussed, the machine learning algorithm might be trained as follows. A training dataset may include training data entries comprising sample outputs from the speech analysis module 62 applied to sample conversations, comprising the detected set of topics/statements for each sample conversation. Each of these may be tagged or annotated in advance with a corresponding score indicating sufficiency of understanding by the patient. The annotations might be derived as follows. For each conversation, the patient could be given a test to determine how well they understood the explanation of the doctor (or a third party observer could rate how well the patient understood). The result of the test, or of the observer rating, could then be used to annotate each training data entry

For the confidence score relating to a confidence that consent has been given by the patient in relation to the consent item, the corresponding training dataset may again include training data entries comprising sample outputs from the speech analysis module applied to sample conversations, comprising the detected set of topics/statements for each conversation. The patient could be asked after each conversation whether they considered that they had consented, or a third-party expert could be asked to assess this. This could then be used to tag or annotate each training data entry according to whether or not the patient actually consented.

Furthermore, in some embodiments, body language and/or facial expressions might additionally be evaluated and linked to parts of the conversation to assess confidence in the patient's comprehension. By way of example, a relaxed posture and/or facial expression can be a sign of comprehension and therefore detection of this state may increase the confidence level. Conversely, a doubtful facial expression, or a patient leaning over towards the physician can be indications of confusion. This may indicate a lower level of confidence in the patient's understanding, and the computation of the score may take this into account.

To interpret the patient's body language and/or facial expressions, use may be made of a dedicated body language and/or facial expression analysis module. By way of example, implementation details may be found in document WO 2019/211220 A1.This document describes how to detect various elements of patient state, including stress, confusion, and level of attention via a combination of physiological measurements, camera-based emotion recognition and speech analysis. The principles and technical implementations discussed in this document could be applied in embodiments of the present invention to detect a patient's state with regards to stress, emotion, and/or attention. This can then be used in determining confidence that the patient has understood well a particular consent item which has been explained to them.

To state the above in more general terms, in some embodiments, the method may further comprise: receiving input camera image data from a camera arranged to record the patient's face and/or body during the conversation, processing the camera image data with a facial expression and/or body language processing module and wherein the determining of the confidence score relating to a sufficiency of understanding by the patient of the consent item discussed further comprises use of an output from the facial expression and/or body language processing module.

In other words body language and/or facial expressions may be evaluated and linked to parts of the conversation to assess confidence in the patient's comprehension.

In accordance with one or more embodiments brain activity patterns could additionally or alternatively be measured to estimate the level of concentration that the patient has on the conversation. A highly focused patient is more likely to correctly understand the conversation than a distracted patient. Brain activity patterns could be recorded for example using an EEG apparatus, and analyzed for example using a suitable algorithm for estimating concentration level. By way of example, reference is made to the following paper: Mohamed Z, El Halaby M, Said T, Shawky D, Badawi A. Characterizing Focused Attention and Working Memory Using EEG. Sensors (Basel). 2018 Nov 2;18(11):3743. doi: 10.3390/s18113743. PMID: 30400215; PMCID: PMC6263653. This describes a suitable method for assessing a person's focused attention based on EEG analysis.

With regards to the confidence score related to the confidence that consent has been given by the patient, this could be performed based on detecting in the conversation portion one or more of a pre-defined list of affirming statements by the patient such as 'yes', 'indeed', 'okay', 'I agree' in relation to the discussion of a consent item. Detection of certain pre-defined gestures, posture and facial expressions could additionally be used. Detection of certain said gestures, posture and facial expressions in combination with the affirmation statements could also be used, e.g. a patient saying 'yes' while nodding.

In a preferred set of embodiments, the method comprises processing the audio data in real time, and providing feedback indicative of results of the consent confidence assessments in real time to the clinician. This allows a clinician to for example supplement the conversation in real time to improve the consent confidence scores.

In some embodiments, if the discussion is linked to a consent item, this triggers a recording of that part of the discussion and storing it (temporarily) in a database in case the doctor needs to or wants to refer back to it later in the conversation. Each such portion of the discussion may in some embodiments be processed in real time, one-by-one.

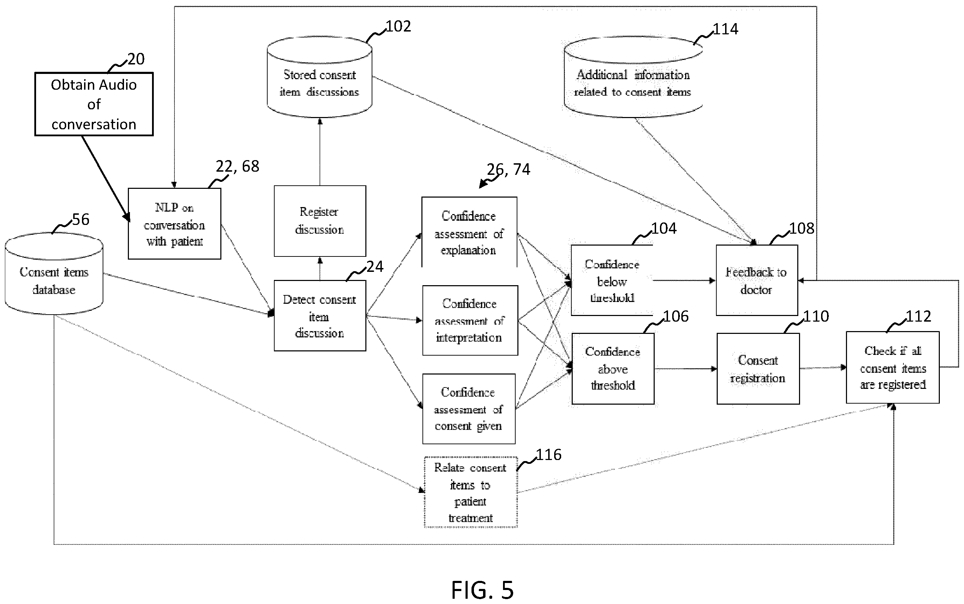

One particular implementation in which real time processing is used will now be described with reference to Fig. 5. It will be appreciated that not all features of this particular set of embodiments are essential to the inventive concept, and are described to aid understanding and to provide an example to illustrate the inventive concepts.

In operation, an audio recording apparatus may be pre-installed in the consultation room of the clinician so that a conversation between the clinician and the patient can be recorded. In this way audio data of the conversation is obtained 20.

As indicated at the box labelled 22, 64 speech-to-text conversion and NLP are applied to the conversation audio to detect when certain consent items are discussed. As has previously been discussed, the speech-to-text conversion can be used to generate a textual representation of the conversation, and NLP can then be used to detect mention in the conversation of various concept, topics or statements.

As indicated in Fig. 5, by application of speech analysis 22, 64 in the manner already discussed previously, the system is configured to detect 24 when at least one of a pre-defined list of consent items is discussed. In a preferred set of embodiments, a speech analysis module is configured to process the audio data in real time and to detect, based on the processing, when discussion of any of the list of consent items begins, and responsive thereto trigger start of a recording of the discussion. The predefined list of consent items might be stored in a consent items database 56 which is accessible by the system. This database contains the individual elements, the separate consent items, of the consent form. When it has been detected that the consent item is being discussed, this may be registered and a corresponding recording of the portion of the conversation relating to that consent item may be stored 102 for later use. For example, it can be stored (temporarily) in a database in case the doctor needs to or wants to refer back to it later in the conversation.

In this set of embodiments, it is also detected when discussion of the given consent item ceases. For example it can be detected that the discussion of the consent item has stopped based on detecting that patient has consented or rejected, or by detecting that the discussion has moved on to another topic. Responsive to detecting that the discussion of the particular consent item has ceased, the system may be configured to stop the recording of the discussion, to provide a recording of the at least portion of the conversation relating to the given consent item. This recording may be stored 102 in a database, as just mentioned.

Following this, the method comprises applying a consent assessment, as indicated at arrow 26, 74. The consent assessment has already been discussed previously, and involves determining at least one, and preferably all, of a set of three confidence scores relating to the consent. A first confidence score relates to sufficiency of explanation by the clinician of the consent item discussed (i.e. the doctor's correct and clear explanation of the consent item). The second confidence score relates to sufficiency and understanding by the patient of the consent item (i.e. the patient's correct interpretation of the consent item). The third confidence score relates to a confidence that consent has been given by the patient in relation to the consent item.

After computation of the consent assessment scores 74, the method may further comprise determining whether each confidence score exceeds a certain predefined threshold for the confidence score. As indicated schematically in Fig. 5, this can include determining whether each confidence score falls below 104 a predefined threshold, and determining whether each confidence score is above 106 a predefined threshold. The results of the comparison with the thresholds might be output to a user interface in some embodiments to provide feedback 108 to the clinician.

In preferred set embodiments, if at least one of the scores falls below its predefined threshold, a feedback loop might be started. Within this feedback loop, an output is generated communication to a user interface the output being based on a result comparison, so as to provide the feedback 108 to the doctor. This feedback can prompt the doctor to expand on or supplement their discussion with further explanation, further questions to assess the patient's understanding, or further questions to prompt the patient to affirm consent, as necessary according to which confidence score is deficient. Following the provision of the feedback 108, the method may comprise repeating the steps of receiving the audio 20 of the conversation, processing it to detect the discussion of consent item, and applying the processing to re-evaluate each the confidence scores. In this way, a feedback loop is established in which the confidence scores are recomputed cyclically until sufficient consent confidence is obtained in respect of each of the scores.

To facilitate this real-time feedback loop, the consent assessment 26, 74, the score comparison 104, 106, and the feedback generation 108 are preferably all triggered immediately responsive to the aforementioned detection of the cessation of the discussion of the given consent item.

In this way, a feedback loop is established wherein the doctor is informed about which of the three confidence assessments is/are below threshold and the doctor is given the opportunity to refer back to the recording of the discussion and to provide more information related to the consent item, to better inform the patient.

Once a consent item has been discussed and all confidence scores exceed the predefined respective thresholds, this may be registered 110 in a datastore, and the process continues with the system analyzing each portion of the conversation relating to each next consent item as it arises in the course of the conversation.

At the end of the conversation, the method may further comprise a step of checking 112 whether all required consent items have been discussed and have been registered 110 as having passed the consent assessment (i.e. that the consent confidence scores have passed their respective thresholds).

In other words, using the language previously introduced, the speech analysis module may be configured to detect an end of the conversation, and wherein the consent assessment further comprises detecting whether consent has not been given for any consent items in the list of consent items in the consent item database 56, and providing feedback to the clinician via a user interface indicative of this detection.

In some embodiments, the method may further comprise, for each consent item for which it has been detected that consent has not been given, retrieving the previously stored recording 102 of the at least portion of the conversation relating to said consent item or the textual representation of the at least portion of the conversation relating to said consent item, and providing via a user interface a control option permitting replaying of the recording, or permitting display of the textual representation.

Thus, feedback at the end of conversation can include parts of the conversation associated with the consent item being recalled by the system and presented back to the doctor and patient for review.

Optionally, in some embodiments, more information 114 on the consent item may be presented by the system to supplement the doctor's explanation of the item. This might be provided as part of the feedback to the doctor 108 along with the results of the consent assessment 26, 74.

As indicated in Fig. 5, in some embodiments, there can be included an additional step 116 in which items listed in the consent items database 56 are refined according to a medical record of the patient so as to identify a subset of the listed consent items that are relevant or related to an upcoming part of the patient's care. The step of checking 112 whether all of the needed consent items have been registered 110 can make use of this refined list in some embodiments. However, this is not essential and instead the list of consent items in the consent items database 56 may be generated so as to be personalized to the particular patient and to the particular element(s) of the patient's care which are relevant for the purposes of the conversation. In yet further embodiments, the list of consent items listed in the consent database 56 may simply be generic consent items, and no personalization to the patient is performed, including no filtering 116 according to the patient record.

In some embodiments, the consent items database 56 could also enable linking of consent items to predicted future treatment of the patient. In other words, in some embodiments, the determining the consent items could be performed partially predictively. For example, it could be partly done through analyzing future appointments or elements of the future care path that can be obtained directly from the patient's medical file. Partly this could be done through predicting the future care path by comparing the patient to similar patients who have already been treated in the past, or by mapping the patient's situation onto an existing protocol or guideline for treatment.

By way of example, this feature could be enabled by creating a knowledge base of all possible diagnoses and treatments, including their care paths (these are typically available for each disease within a medical institution), and linking them to the relevant consent items.

Such a knowledge base could be created by translating expert knowledge into a formal rule base, e.g. by having experts label/annotate different treatments with relevant consent items. In another embodiment, this can be done automatically, based on semantic comparison of available care paths/protocols with consent items.

Furthermore, to enable this, in some embodiments, the method comprises mapping out the patient's care path. This can be partly done through analyzing future appointments or elements of the future care path that can be obtained directly from the patient's medical file. Partly this can be done through predicting the future care path by comparing the patient to similar patients who have already been treated in the past, or by mapping the patient's situation onto an existing protocol or guideline for treatment.

For example, the method may further comprise communicating with a patient database, accessing a patient record for the patient, wherein the patient record includes an indication of one or more planned elements of patient care for the patient, and wherein the method further comprises generating the list of consent items in the consent item database 56 based on application of a lookup table which maps elements of patient care to consent items.

In some embodiments, the system may further keep track of consent that has already been obtained in conversations with other health care professionals. Feedback may be generated to communicate to the doctor in the current conversation which items do not require consent anymore and which do, or the list of consent items may be generated or refined based on this information.

For example, in some embodiments, the method further comprises communicating with a patient consent database storing a record of consent items for which consent has been given by the patient, and wherein the list of consent items is modified to remove any consent items for which said patient consent database indicated consent has already been given.

For example, in some embodiments, the consent registration is stored in a personal health record of the patient, or some other centrally stored record, such that it can be shared between care institutes.

Furthermore, in some embodiments, the method may further comprise communicating with a patient consent database storing a record of consent items for which consent has been given by the patient, and updating the database to reflect consent items for which the consent assessment is positive.

In some embodiments, said patient consent database may be a centrally accessible database, accessible by multiple care institutions.

In some embodiments, in addition to the feedback relating to consent items for which the consent assessment is not yet positive, feedback may be generated for the doctor which is indicative of guidance for structuring the conversation. For example it may comprise guidance for the doctor in selecting which consent items should be discussed, and optionally in which order, given the patient's planned or predicted future care path.

For example, this could take the form of a list of consent items, in which text color or one or more other display properties of the text is adjusted in real time depending upon which have been discussed already, which have been discussed but do not meet one or more of the confidence score thresholds, and those which have been discussed and for which the confidence assessment is positive (possibly also with an indication of the confidence scores).

Various embodiments make use of a user interface device. With regards to the user interface, this preferably comprises a display. It may comprise a user input means. It may comprise or be fulfilled by a portable computing device such as a tablet computer or a smartphone. It may be implemented in some examples using an augmented reality headset, to minimally hamper the conversation with the patient.

Embodiments of the invention described above employ a processing unit. The processing unit may in general comprise a single processor or a plurality of processors. It may be located in a single containing device, structure or unit, or it may be distributed between a plurality of different devices, structures or units. Reference therefore to the processing unit being adapted or configured to perform a particular step or task may correspond to that step or task being performed by any one or more of a plurality of processing components, either alone or in combination. The skilled person will understand how such a distributed processing unit can be implemented. The processing unit includes a communication module or input/output for receiving data and outputting data to further components.

The one or more processors of the processing unit can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A computer-implemented method (10), comprising:
communicating with a datastore to access (18) a data list reciting a plurality of consent items, each consent item corresponding to an element of patient care which requires patient consent;
receiving (20) audio data of the whole or part of a patient-clinician conversation;
processing the audio data with a speech analysis module, wherein the speech analysis module is configured to:
apply automated speech recognition to convert (22) the audio data to a textual representation of the conversation; and
apply natural language processing to the textual representation of the conversation to detect (24) at least a portion of the conversation in which at least one of the consent items is discussed; and
for each said at least portion of the conversation, apply (26) a consent assessment comprising applying natural language processing to determine one or more of: a confidence score relating to a sufficiency of explanation by the clinician of the consent item discussed; a confidence score relating to a sufficiency of understanding by the patient of the consent item discussed; and a confidence score relating to a confidence that consent has been given by the patient in relation to the consent item.

2. The method of claim 1,
wherein the speech analysis module is adapted to apply natural language processing to the textual representation of the conversation to detect discussion or presence in the conversation of one or more of a pre-defined list of topics and/or statements recorded in a topic database; and
wherein the topic database further records for each of the pre-defined list of topics and/or statements one or more of the consent items to which the topic and/or statement is relevant.

3. The method of claim 2,
wherein the method comprises relating each of the topics and/or statements detected in the conversation to one or more of the consent items using the topic database; and
wherein determining the one or more confidence scores for a given consent item and/or determining the at least portion of the conversation in which at least one of the consent items is discussed is performed based on analysis of the detected topics and/or statements which have been detected as related to the given consent item.

4. The method of claim 3, wherein the one or more confidence scores are determined based on evaluation of one or more pre-defined criteria relating to the topics and/or statements detected as relevant to the given consent item.

5. The method of claim 3 or 4, wherein the determining the one or more confidence scores is performed based on counting the number of topics and/or statements which have been detected as related to the given consent item for which the score is being determined.

6. The method of any of claims 3-5, wherein determining the confidence score relating to a sufficiency of explanation includes:
counting how many of a pre-defined list of topics and/or statements relating to the given consent item are present in the at least portion of the conversation; and/or
mapping of detected topics and/or statements present in the at least portion of the conversation to one or more logic rules to assess whether the one or more logic rules are met.

7. The method of any of claims 1-6, wherein the method comprises processing the audio data in real time, and providing feedback indicative of results of the consent confidence assessments in real time to the clinician.

8. The method of any of claims 1-7,
wherein the speech analysis module is configured to process the audio data in real time and to detect, based on the processing, when discussion of any of the list of consent items begins, and responsive thereto trigger start of a recording of the discussion.

9. The method of claim 8, wherein, following start of the recording, the speech analysis module is configured to detect, based on the processing, when the discussion of the given consent item ceases, and responsive thereto:
stop the recording of the discussion, to provide a recording of the at least portion of the conversation relating to the given consent item; and
apply the consent assessment to said at least portion of the conversation covered by the recording.

10. The method of claim 9,
wherein the method comprises comparing each of the one or more confidence scores of the consent assessment against a respective threshold, and
generating an output for communication to a user interface, the output being based on a result of the comparison; and
optionally wherein the consent assessment, the score comparison and the output generation are triggered immediately responsive to said detection of the cessation of the discussion of the given consent item of the at least portion of the conversation relating to the given consent item.

11. The method of any of claims 1-10, wherein the speech analysis module is further configured to detect an end of the conversation, and wherein the consent assessment further comprises detecting whether consent has not been given for any consent items in said list of consent items, and providing feedback to the clinician via a user interface indicative of this detection.

12. The method of claim 11 when dependent on claim 9, wherein the method further comprises, for each consent item for which it has been detected that consent has not been given:
retrieving the recording of the at least portion of the conversation relating to said consent item or the textual representation of the at least portion of the conversation relating to said consent item,
providing via a user interface a control option permitting replaying of the recording, or permitting display of the textual representation.

13. The method of any of claims 1-12, wherein the method further comprises communicating with a patient database, accessing a patient record for the patient, wherein the patient record includes an indication of one or more planned elements of patient care for the patient, and wherein the method further comprises generating said list of consent items based on application of a lookup table which maps elements of patient care to consent items.

14. A computer program product comprising code means configured, when run on a processor, to cause the processor to perform a method in accordance with any of claims 1-13.

15. A processing unit comprising:
an input/output; and
one or more processors adapted to:
communicate, via the input/output, with a datastore to access a data list reciting a plurality of consent items, each consent item corresponding to an element of patient care which requires patient consent;
receive audio data of the whole or part of a patient-clinician conversation;
process the audio data with a speech analysis module, wherein the speech analysis module is configured to:
apply automated speech recognition to convert the audio data to a textual representation of the conversation; and
apply natural language processing to the textual representation of the conversation to detect at least a portion of the conversation in which at least one of the consent items is discussed;
for each said at least portion of the conversation, apply a consent assessment comprising applying natural language processing to determine one or more of: a confidence score relating to a sufficiency of explanation by the clinician of the consent item discussed; a confidence score relating to a sufficiency of understanding by the patient of the consent item discussed; and a confidence score relating to a confidence that consent has been given by the patient in relation to the consent item.
